# EUROPEAN PATENT SPECIFICATION

(11) **EP 1 438 964 B1**
(45) Date of publication and mention of the grant of the patent: **19.03.2008**
(21) Application number: 02762761.1
(22) Date of filing: 08.08.2002
(51) Int. Cl.: A61K 35/64, A61K 31/375, A61K 8/00, A23L 1/302, A23L 1/076, A23K 1/16, A61P 43/00

(54) **PROCESS FOR PRODUCING COLLAGEN PRODUCTION ENHANCERS AND USE THEREOF**
VERFAHREN ZUR HERSTELLUNG VON KOLLAGEN-PRODUKTIONSVERSTÄRKERN UND VERWENDUNG
PROCEDE DE PRODUCTION D'ACTIVATEURS DE PRODUCTION DE COLLAGENE ET UTILISATION DE CES ACTIVATEURS

(30) Priority: 27.09.2001 JP 2001295464; 10.07.2002 JP 2002201883
(43) Date of publication of application: 21.07.2004
(73) Proprietor: KABUSHIKI KAISHA HAYASHIBARA SEIBUTSU KAGAKU KENKYUJO, Okayama-shi, Okayama 700-0907 (JP)
(72) Inventor: MIYATA, Satomi c/o K.K. Hayashibara, Okayama-shi, Okayama 700-0907 (JP); USHIO, Shinpei c/o K.K.Hayashibara, Okayama-shi, Okayama 700-0907 (JP); KURIMOTO, Masashi c/o K.K.Hayashibara, Okayama-shi, Okayama 700-0907 (JP)
(74) Representative: Daniels, Jeffrey Nicholas
(86) International application number: PCT/JP2002/008133
(87) International publication number: WO 2003/028744

(56) References cited:
- WO-A1-00/09121
- WO-A1-00/51561
- WO-A1-01/60388
- WO-A1-96/38168
- JP-A- 7 184 596
- JP-A- 11 266 832
- JP-A- 2000 069 912
- JP-A- 2000 109 417
- JP-A- 2000 189 125
- JP-A- 2001 240 529
- JP-A- 2002 226 355
- TSUTSUMI, KISHIDA, HATA: 'Mitsubachi yurai genryo no kashohin eno oyo' FRAGRANCE JOURNAL vol. 30, no. 3, March 2002, pages 17 - 24, XP002963464

## Description

### Technical Field

The present invention relates to a novel collagen-production enhancer, particularly, to a collagen-production enhancer comprising L-ascorbic acid and/or the like (designated as "L-ascorbic acids" hereinafter, unless specified otherwise) and royal jelly and/or the like (designated as "royal jellies" hereinafter, unless specified otherwise); and a composition comprising the enhancer.

### Background Art

JP2000-189125A discloses an acidic drinking liquid composition containing (A) 0.001-0.01 wt.% of one or more than two kinds of vitamin B1 compound (derivative) -selected from dibenzoylthiamin hydrochloride, thiamin naphthalene-1,5-disulfonate or bisbenthiamin and (B) 0.1-0.3 wit.% of ascorbic acid (salt), and further preferably (C) one or more than two kinds of crude drug extract selected from American carrot, Acanthopanax senticosus, polygonatum sibiricum Red., Panax ginseng, Guarana, Chinese matrimony vine, Panax notoginseng, Cordyceps sinensis, Acanthopanax sieboldianus, Fomes japonicus, royal jelly and the like and has pH 2.5-4.0.

WO01/60388A1 discloses a cell activator which contains royal jelly and trehalose.

JP11266832A discloses a mixed food composition obtained by blending a food composition comprising at least a royal jelly, &beta-carotene, ascorbic acid, tocopherol and rice bran extract with a food composition comprising at least a spirulina, protamine, beer yeast and lactic acid in the weight ratio of (1 to 0.1) to (1 to 10).

JP2000-109417A discloses a cosmetic composition obtained by mixing (A) an elastase activity inhibitor, suitably a soybean protein and/or an essence of Engelhardtia chrysolepis H, with (B) a melanin production suppressant, suitably one or more than two kinds selected from arbutin, vitamin C, a vitamin C derivative, an essence of royal jelly and an essence of mulberry bark.

L-Ascorbic acid or vitamin C is an essential nutritional element that is not produced *in vivo* in humans, monkeys, and guinea pigs. The compound is known to have efficacy in the prevention and treatment of scurvy, and in fact it correlates with *in vivo* physiological actions such as the production of collagen as a main ingredient of connective tissues and to the immuno-enhancement action via the enhancement of leukocyte production, and plays an important role in the maintenance/promotion of the health of living bodies. Not only as an essential nutritional element, L-ascorbic acid is widely used in food products as an acid agent, pH-controlling agent, antioxidant, browning preventive, etc.; preventives and remedies for diseases such as viral, bacterial, and malignant diseases; and in cosmetics such as skin-beautifying and skin-whitening agents such as ultraviolet absorbents and melanin-formation inhibitory agents. L-Ascorbic acid is unstable and susceptible to oxidation due to its direct reducibility, and this easily causes the reduction of its physiological activities. To improve such defect, L-ascorbic acid derivatives such as L-ascorbic acid glucosides, L-ascorbic acid phosphates, and L-ascorbic acid sulfates were explored as stabilized compounds of L-ascorbic acid. Since such L-ascorbic acid derivatives are produced from material L-ascorbic acid, the production costs inevitably become higher than that of the material L-ascorbic acid. Considering the use of L-ascorbic acid and/or derivatives thereof, they are not satisfactory because, when administered in a relatively large amount, they may cause a temporal disorder to the skin and the oral and gastrointestinal mucosae due to their strong acidity and form a quantity of metabolites of L-ascorbic acid. Further, from a view point of economical benefit, required are the products such as daily usable health foods, which can exert sufficient physiological actions of L-ascorbic acid even when administered in a lesser amount.

Royal jelly is a milky-white secretion from the external gland of worker bees, which is accumulated in royal cell, i.e., a queen bee's cluster, and which is a feed for a specific bee larva to become a queen bee. The larva hatched in the royal cell could not be distinguished from other bees at that time but becomes to a fertile queen bee distinguishable from other worker bees in size and life time after growth on sufficient royal jelly. Based on the fact, royal jelly has been expected to have a variety of physiological actions such as tonic- and sexual potential-actions, and it has been actually known by experience to have the above-identified actions and used as health foods from a long time ago. Recently, many technical literatures have been reported in terms of the antiseptic, immune-enhancement, antitumor, and anti-inflammatory actions of royal jelly. Since royal jelly is a natural product, it is understood that it does not cause any serious side effect when administered to humans and other animals. Therefore, royal jelly has been widely used as a material for health foods and cosmetics.

As described above, L-ascorbic acid and royal jelly have been extensively used as materials for health foods and cosmetics. Examples of such are food products comprising L-ascorbic acid and royal jelly, disclosed in Japanese Patent Kokai Nos. 2000-342331 and 2000-60455; skin external applications comprising L-ascorbic acid and royal jelly, disclosed in Japanese Patent Kokai No.93706/88; and skin external applications comprising an L-ascorbic acid derivative and royal jelly, disclosed in Japanese Patent Kokai No. 2000-63226.

However, there is found neither report on the enhancement of collagen production by royal jelly nor one on the finding that royal jelly enhances the collagen production by L-ascorbic acid.

After coming into this aging society, the changes in body conditions such as age-related reduction of the thickness and the metabolism of the skin are troublesome for most of the middle and senior generations, particularly, for women. Representatives of such are facial changes accompanied by distinctively noticed facial wrinkles/fine-wrinkles, liver spots, facial skin sagging, loss of skin gloss and fitness, and reduction of skin elasticity. Cosmetics, which are supplemented with collagen and mucopolysaccharides such as hyaluronic acid for imparting humectancy to the skin, have been conventionally explored to prevent the skin aging, but they could not yet attain a sufficient effect on such purpose. Recently progressed researches on the skin aging revealed that a remarkable reduction of the level of collagen fibers as a constituent for the dermis is a major causative of the skin aging. It has been also indicated that the reduction of collagen fibers correlates with the facial changes such as the reduction of facial wrinkles/finely-wrinkles, liver spots, and gloss; the loss of skin fitness; the occurrence of facial skin sagging; and the reduction of skin elasticity. Although there exist many causatives of the skin aging, it finally eventuates in both the reduction of metabolism of collagen through the reduction of collagen-production ability of fibroblasts in the dermis, and the reduction of proliferation ability of fibroblasts *per se.* Since the skin has the epidermis, that are composed of keratinocyte, outside the dermis, the reduction of the function of keratinocytes weakens the keratinocyte layer on the skin surface, slows the regeneration rate of the layer, and lowers the biophylactic ability as a function inherent to the skin. Accordingly, factors including bacterial infections that are responsible for the above functional reduction would accelerate the damage to fibroblasts in the dermis and throughout the skin, resulting in a more accelerated skin aging. The reduction of the collagen production in such fibroblasts accelerates not only the skin aging but also weakens vasculars, as well as tissues and organs in living bodies, whose structures are maintained by collagen; but also affects healthy conditions of the living bodies. Since collagen, a kind of protein, is not substantially absorbed by living bodies directly if only orally taken or extradermally applied to the skin and it could not augment the activity of fibroblasts and keratinocytes, collagen *per se* could not be a primary preventive/therapeutic for the skin aging. In order to prevent the skin aging and to maintain/promote the health, it is desired to explore agents for enhancing the collagen production that is safe and capable of continuously augmenting the production of collagen as a main constituent for dermis, tissues, and organs; and agents for activating fibroblasts *per se* existing in the dermis constituting the skin; and keratinocytes *per se* existing in the epidermis.

Under these circumstances, the object of the present invention is to provide a means for effectively enhance the production of collagen by L-ascorbic acids.

### Disclosure of Invention

To overcome the above object, the present inventors repeatedly made researches and screenings using fibroblasts and resulted in an unexpected finding that, when royal jellies are added, the production of collagen by L-ascorbic acids is effectively augmented even when used at a concentration, where any such production could not be observed or merely slightly observed in a single use of L-ascorbic acids. As a result, the present inventors reached a self-finding that the coexistence of royal jellies augments the production of collagen even when L-ascorbic acids are in a condition of being inactivated up to be incapable of inducing the production of collagen by itself. While it has been known that the production of collagen in fibroblasts is augmented by transforming growth factor (abbreviated as "TGF-β" hereinafter) secreted from fibroblasts, etc. Further, the present inventors focused on this and continued studying, revealing that royal jellies act on fibroblasts and augment the production of TGF-β in the presence of L-ascorbic acids, and that royal jellies *per se* act on keratinocytes existing in the surface side of the skin rather than the dermis and augment the production of TGF-β. Thus, they confirmed that one of the mechanisms of the enhancement of the collagen production by L-ascorbic acids, that is induced by royal jellies, is the augmentation of the production of TGF-β, and that royal jellies in combination with or without L-ascorbic acids are useful as an agent for enhancing the production of collagen or TGF-β, and/or an agent for promoting the proliferation of keratinocyte. Thus, the present inventors accomplished this invention.

The present invention solves the above object by providing a composition for enhancing the production of collagen or TGF-β in fibroblast, which comprises L-ascorbic acid or derivative thereof in an amount of at least 0.02% by weight and royal jelly in an amount of at least 0.5 part by weight to one part by weight of said L-ascorbic acid or said derivative thereof. The present invention also provides use of this composition for the manufacture of a medicament - for enhancing the production of collagen.

The present invention further provides use of royal jelly for the manufacture of a medicament for enhancing TGF-β production, use of royal jelly for the manufacture of a medicament for promoting the proliferation of keratinocytes, and use of royal jelly for the manufacture of a medicament for enhancing the production of collagen or TGF-β in fibroblasts by L-ascorbic acid or a derivative thereof.

### Best Mode for Carrying Out the Invention

The collagen-production enhancer of the present invention comprises L-ascorbic acid and/or the like(s), i.e., L-ascorbic acids, and royal jelly and/or the like(s), i.e., royal jellies. The term "L-ascorbic acids" as referred to as in the present invention means those which exert the physiological functions of L-ascorbic acid *in vivo* and one or more of the following compounds can be used in combination, for example, those in the form of a composition or mixture: L-Ascorbic acid and/or salts thereof, and L-ascorbic acid derivatives and/or salts thereof including L-ascorbic acid 2-glycosides such as L-ascorbic acid 2-glucoside; and others such as L-ascorbic acid phosphates, L-ascorbic acid sulfates, DL-α-tocopherol 2-L-ascorbic acid phosphoric acid diesters, and acyl derivatives such as L-ascorbic acid 2-glucoside. The term "salt(s) of L-ascorbic acid" as the L-ascorbic acids as referred to as in the present invention means any one or an appropriate combination of inorganic and/or organic salts of L-ascorbic acid such as sodium-, potassium-, calcium-, magnesium-, ammonium-, and alkylammonium-L-ascorbates. Since stable derivatives of L-ascorbic acid such as L-ascorbic acid 2-glycosides, which belong to L-ascorbic acids, are substantially free from oxidative decomposition even when administered to the skin and gradually decomposed by *in vivo* enzymes to exert a controlled-releasing effect, they can be reduced their administration frequency and exert the effects inherent to L-ascorbic acid for a longer period of time as compared with L-ascorbic acid when administered to living bodies. Thus, the stable derivatives of L-ascorbic acid can be particularly desirable as the L-ascorbic acids used in the collagen-production enhancer of the present invention.

Although concrete experimental data are not shown in the present specification, the present inventors confirmed that all the above-identified ascorbic acids were absorbed in the form of L-ascorbic acid by animals and humans and exerted the collagen-production action, independently of their oral intake or transdermal administration. When applied to feeds, baits for fish, pet foods, etc., for animals capable of synthesizing L-ascorbic acid *in vivo,* L-glono-γ-lactone as a precursor of L-ascorbic acid, that is converted into L-ascorbic acid in such animals, can be used as the ascorbic acids in the present invention.

The royal jelly as referred to as in the present invention means a milky white liquid substance, secreted from worker bees and accumulated in a royal cell, that is fed to a larva to become a queen bee. The term "royal jellies" as referred to as in the present invention means intact royal jelly or compositions produced therefrom as a material and includes those in a natural liquid form or those in an artificially processed liquid form or other forms of a solid, powder, granule, paste, etc., as long as they enhance the collagen production by L-ascorbic acid. The royal jelly should not specifically be restricted to the kinds of bees to produce it and the sources/regions thereof. Examples of the kind of such bees include those of the species *Apis mellifera, Apis cerana*, *Apis dorsata,* and *Apis florea.* Examples of the sources/regions of the royal jelly are Japan, South America, North America, Australia, China, and Europe. In general, royal jelly could induce unfavorable side effects such as allergic reactions depending on subjects to be administered. Since those produced from South America, more preferably, those from Brazil are relatively low in such side effects, the latter is particularly advantageously used in practicing the present invention. As disclosed in Japanese Patent Kokai No. 9,457/85, intact/fresh royal jelly (designated as "unheated royal jelly" hereinafter) is deteriorated by heating so that it should generally be avoided from heating at temperatures of 65°C or over. Since even a royal jelly, heated at a temperature of 70°C or over for at least 30 min (designated as "heated royal jelly" hereinafter), retains the action of enhancing the production of royal jelly by L-ascorbic acid, heated royal jelly, which has been sterilized by heating or heated to denature allergic proteinaceous ingredients to lower or even eliminate their allergenicity, can be advantageously used as the materials in the present invention. In addition to the above-mentioned heated and unheated royal jellies, the royal jellies usable in the present invention include any of those which are prepared by partially purifying the above heated and unheated royal jellies with extractions using solvents such as acetone, ethanol and water; gel filtration; and other appropriate methods; and which have the action of enhancing the collagen production by L-ascorbic acid. Hereinafter, throughout the specification, the aforesaid heated and unheated royal jellies are simply called "royal jelly", unless specified otherwise.

The collagen-production enhancer of the present invention comprises the above-mentioned L-ascorbic acids and royal jellies. Referring to the amount of L-ascorbic acids and royal jellies incorporated in the collagen-production enhancer of the present invention, it is preferably at least minimum level requisite for enhancing the collagen production by L-ascorbic acids using royal jellies. The action of enhancing the collagen production by L-ascorbic acids using royal jellies can be assayed by the method in the later-described experiment for quantifying the produced collagen in fibroblasts prepared from a new born infant hamster. The collagen-production enhancer of the present invention contains at least 0.02% by weight of L-ascorbic acids in terms of L-ascorbic acid, preferably, at least 0.05% by weight to the total weight of the enhancer; and at least 0.5 part by weight, preferably, at least one part by weight of royal jellies to one part by weight of the L-ascorbic acids in terms of L-ascorbic acid.

The collagen-production enhancer of the present invention optionally comprises an antioxidant(s). Examples of such an antioxidant(s) include those which can inhibit the oxidative decomposition of the L-ascorbic acids contained in the collagen-production enhancer during storage. Such antioxidants attain a higher level of stabilization of the collagen-production enhancer. In the case of using the collagen-production enhancer of the present invention in foods for animals and humans, appropriate antioxidants can be selected from those which are generally used in the field of foods. For example, flavonoids, polyphenols, and vitamin E are advantageously used as the antioxidants in the present invention. The amount of these antioxidants to be incorporated in the collagen-production enhancer of the present invention is not specifically restricted, however, from a view point of taste, it is preferably set to a level according to those which are generally used in the field of foods or to a level slightly lower than those. While in the case of applying the collagen-production enhancer of the present invention to the fields of cosmetics, quasi-drugs, and pharmaceuticals, the antioxidants generally used in such fields can be incorporated in the enhancer in an amount according to those generally used in such fields or at a level slightly lower than those.

To the collagen-production enhancer of the present invention can be added one or more of the following ingredients as fillers when in a solid form, and they can be added, for example, to stabilize, improve taste, and retain flavor and taste of the enhancer: Saccharides such as glucose, fructose, lactose, trehalose, maltose, sucrose, lactosucrose, and starch hydrolyzates; saccharides with cyclic structures such as cyclodextrins and cyclotetrasaccharide disclosed in International Patent Publication Number WO 02/10361, titled "α-Isomaltosylglucosaccharide-forming enzyme, its preparation and uses" applied for by the same applicant as the present invention; sugar alcohols such as erythritol, mannitol, sorbitol, xylitol, maltitol, and hydrogenated starch syrup; sweeteners with high sweetness such as aspartame, stevia extracts, sucralose, and acesulfame K; polysaccharides such as pullulan and carrageenan; and thickening agents or viscosity-imparting agents such as natural gums and synthetic carboxymethyl cellulose.

In addition to the royal jellies and L-ascorbic acids, for example, ingredients having an action of enhancing collagen production, which are extracted from buds of *Fagus engleriana Seemen* disclosed in Japanese Patent Kokai No. 203,952/98, can be optionally incorporated into the collagen-production enhancer of the present invention. If necessary, one or more of emulsifiers, flavors, spices, pigments/dyes, amino acids, and vitamins other than L-ascorbic acid such as vitamins B₁, B₂, B₆, E, and P, and derivatives thereof can be incorporated. Usually, the above-identified ingredients can be appropriately selected based on the criterion of the need in the fields to which the collagen-production enhancer is applied. The collagen-production enhancer of the present invention, which optionally comprises one or more of the above ingredients, should not be restricted to have a specific form and can be provided in the desired form of a powder, granule, tablet, paste, jelly, milky lotion, or solution.

The effective ingredients in the collagen-production enhancer of the present invention are smoothly absorbed by humans and animals independently of its administration routes such as oral and transdermal administrations to continuously enhance the collagen production by L-ascorbic acid in fibroblasts in the dermis, tissues, and organs. When taken by humans and animals, the collagen-production enhancer exerts the following effects: It stably retains the collagen production, recovers the akin with a reduced collagen-production ability responsible for age-related senility and factors such as ultraviolet ray, imparts an adequate fitness and moisture to the skin, removes fine-wrinkles, and recovers the elasticity of the skin. When transdermally administered, L-ascorbic acids and royal jellies smoothly reach fibroblasts in the dermis and the keratinocytes in the epidermis and enhance the production of TGF-β, resulting in a continuous enhancement of the collagen production by L-ascorbic acids and a promotion of the keratinocyte proliferation by royal jellies to strengthen the keratinocyte layer and to enhance biophylaxis. Thus, L-ascorbic acids and royal jellies smoothly recover the reduction of the collagen productivity of the skin responsible for age-related senility or of the skin damaged by factors such as ultraviolet ray and harmful bacteria, impart an adequate moisture level to the skin, remove fine-wrinkles, and quite effectively recover the elasticity of the skin. The collagen-production enhancer of the present invention is arbitrarily applicable to humans and animals in the maintenance/promotion of health, and it can be specifically used as a tonic, TGF-β production enhancer, keratinocyte-proliferation enhancer, health food, supplemental health food, food for special dietary uses, healthy functional food, cosmetic, quasi-drug, pharmaceutical, feed, baits for fish, pet food, other daily goods, etc.

Varying depending on the kind, age, gender, etc., of humans and animals including pet animals to be administered, the intake amount or the dose of the collagen-production enhancer of the present invention is usually from 0.1 mg to 0.25 g, preferably, from 1 mg to 0.5 g/kg body of a subject in terms of the weight of L-ascorbic acid; and it is usually from 0.5 mg to 2 g, preferably, from 1 mg to 1 g/kg body of a subject in terms of royal jelly at a frequency of once or several times a day when orally taken or administered for successive days or at an interval of one or more days. Referring to the collagen-production enhancer as an orally-administrable tonic, health food, supplemental health food, food for special dietary uses, healthy functional food, quasi-drug, pharmaceutical, feed, baits for fish, pet food, etc., they can be used in the form of a liquid, tablet, powder, granule, paste, syrup, capsule, etc., depending on their uses. In the case of directly applying the collagen-production enhancer of the present invention to the skin as an external dermatological application such as cosmetics, the L-ascorbic acids and royal jellies can be incorporated thereinto at a concentration of 0.001 to 20% by weight, preferably, 0.005 to 15% by weight in terms of L-ascorbic acid or royal jelly to the total weight of the application, and can be directly administered to the skin at a frequency of once or several times a day for successive days or at an interval of one or more days. At a concentration of less than 0.001% by weight of the L-ascorbic acids or the royal jellies, the desired effect could not be attained, while at a concentration of over 30% by weight, it may spoil the property of products containing them. The above-mentioned dermatological application can be prepared into the desired form to meet its final use, for example, those in the form of a lotion, milky lotion, cream, solid, powder, jelly, pack, or face mask.

The collagen-production enhancer of the present invention in itself has advantages as mentioned above, and it can be arbitrarily used in the form of a composition with other ingredient(s). To produce compositions comprising the collagen-production enhancer of the present invention, L-ascorbic acids and royal jellies can be mixed together with one or more ingredients used in the fields of above-mentioned foods, cosmetics, quasi-drugs, pharmaceuticals, feeds, baits for fish, pet foods, etc., in an appropriate amount for each ingredient, according to appropriate compositions selected depending on animals to be administered and on the intake/administration methods; and optionally, based on the content of each of the above ingredients, treated with the following steps of dilution, concentration, drying, filtration, centrifugation, etc., to meet its final use. Then, the compositions thus obtained can be shaped into the desired forms. The order of adding the above ingredients and the timing of applying the above steps are not specifically restricted as long as they should not deteriorate the L-ascorbic acids and royal jellies. For example, unheated royal jellies, which are as fresh as possible or have been preserved under cold conditions after harvest, are mixed with L-ascorbic acids, and optionally, appropriately treated with the above steps. When the collagen-production enhancer of the present invention is prepared with heated royal jellies, L-ascorbic acids should preferably be incorporated after the heat treatment of unheated royal jellies because L-ascorbic acids are unstable to heat. The ingredients usable in the present invention, which are orally, percutaneously, or transdermally applicable to humans and animals, include those which are usually used in each field of the compositions of the present invention used, for example, water, alcohols, amylaceous substances, proteins, amino acids, fibers, saccharides, lipids, fatty acids, vitamins, minerals, flavors, colors, sweeteners, seasonings, spices, aseptics, emulsifiers, and surfactants. The compositions of the present invention can be advantageously used in the fields of foods and beverages including those of animal feeds, baits for fish, and pet foods; foods for special dietary uses; healthy functional foods; cosmetics; quasi-drugs; pharmaceuticals; and daily goods.

The collagen-production enhancer in the form of a solid according to the present invention can be prepared, for example, by mixing L-ascorbic acids with unheated royal jellies, and optionally further mixing with other ingredients and then subjecting the resulting mixture to conventional drying steps such as drying under reduced pressure, drying *in vacuo,* and drying with hot air. By using, for example, as a filler, crystalline or amorphous α,α-trehalose anhydride disclosed in Japanese Patent Kokai No. 170,221/94, applied for by the same applicant as the present invention, the above-mentioned collagen-production enhancer in the form of a solid can be obtained without the need of any conventional drying step; such a solid collagen-production enhancer can be prepared by adding crystalline or amorphous α,α-trehalose anhydride to a mixture of L-ascorbic acids and unheated royal jellies, and allowing the resulting mixture to stand at ambient temperature or lower. The products, dried with α,α-trehalose anhydride without using any conventional drying step, can be suitably used in the present invention because they have an action of enhancing the collagen production by L-ascorbic acid using unheated royal jellies, and have a particularly superior activity of stabilizing the actions of unheated royal jellies. The above-mentioned collagen-production enhancer in the form of a solid can be optionally shaped into the desired form of a powder, granule, or tablet using a pulverizer, granulator, or tabletting machine; and if necessary the resulting powers or granules can be arbitrarily injected into capsules for use. Any desiccants can be used to pulverize the collagen-production enhancer of the present invention as long as they are edible and capable of dehydrating royal jellies while stably retaining their activities. Preferable examples of such are anhydrous α,β-trehalose, anhydrous maltose, anhydrous or monohydrous cyclic tetrasaccharides, and anhydrous α,α-trehalose.

The compositions of the present invention are not restricted to specific ones. Preferred examples of the food compositions with the collagen-production enhancer of the present invention are frozen desserts such as an ice cream, ice candy, and sherbet; syrups such as "*korimitsu*" (a sugar syrup for shaved ice); spreads and pastes such as a butter paste, custard cream, flour paste, peanut paste, and fruit paste; Western cakes such as a chocolate, jelly, candy, gummy jelly, caramel, chewing gum, pudding, cream puff, and sponge cake; processed fruits and vegetables such as a jam, marmalade, "*syrup-zuke*" (a fruit pickle), and "*toka*" (a conserve); Japanese cakes such as "*manju*" (a bun with a bean-jam), "*uiro*" (a sweet rice jelly), "*an*" (a bean jam), "*yokan*" (a sweet jelly of beans), "*mizu-yokan*" (a soft adzuki-bean jelly), *pao de Castella,* "*amedama*" (a Japanese toffee), and rice cake; and seasonings such as a soy sauce, powdered soy sauce, "*miso*", "*funmatsu-miso*" (a powdered *miso*), mayonnaise, dressing, vinegar, "*sanbai-zu*" (a sauce of sugar, soy sauce and vinegar), table sugar, and coffee sugar. Preferable examples of the compositions with the collagen-production enhancer in the form of a food are alcohols such as a synthetic *sake*, fermented liquors including *sake*, wine, and liquors; soft drinks such as a juice, mineral beverage, carbonated beverage, sour milk beverage, lactic acid bacteria drink, isotonic drink, nutritious supplement drink, teas including green tea, tea, and oolong tea, and other beverages including coffe and cocoa. Preferable examples of the compositions with the collagen-production enhancer in the form of a cosmetic are basic cosmetics, face-wash cosmetics, bath cosmetics, oral cosmetics, sun-burn/sun-tan cosmetics, make-up cosmetics, hair cosmetics such as a baldness remedy and hair restorer, and daily goods such as kitchen detergents that directly affect the skin, which are in the form of a lotion, milky lotion, cream, solid, powder, jelly, pack, or face mask. To produce the above-identified compositions, the collagen-production enhancer of the present invention can be added to, or L-ascorbic acids can be added separately from royal jellies to the objective final products at an appropriate timing during their production processes according to conventional methods. The timing for such addition should not be restricted, however, when the objective products are produced through a heating step, L-ascorbic acids and other thermally labile ingredients should preferably be added at ambient temperature, preferably, at temperatures of not higher than 30°C after the heating step, whereby the reduction of the activity of the collagen-production enhancer can be prevented during processings of the compositions. The above-identified compositions according to the present invention usually contain 0.01 to 20% by weight, preferably, 0.1 to 10% by weight of the collagen-production enhancer of the present invention to the total weight of the compositions each.

In the case of using L-ascorbic acid derivatives such as L-ascorbic acid 2-glycosides as the L-ascorbic acids in the present invention, such derivatives are hydrolyzed by enzymes present *in vivo* and/or on the cell surface of living bodies, etc. While in the case of using L-ascorbates such as potassium L-ascorbate are dissociated into ionized forms thereof, and then the resulting L-ascorbic acid from the administered L-ascorbic acids will be transferred to fibroblasts in the dermis and organs and to keratinocytes in the epidermis. In parallel, royal jellies, that are absorbed by living bodies simultaneously with the L-ascorbic acids, act on fibroblasts and/or keratinocytes and enhance the production of TGF-β, resulting in sustainably facilitating the action of enhancing the collagen production. Similarly as conventional daily-use products, daily use of the collagen-production enhancer of the present invention for living bodies will effectively exert the action of enhancing the collagen production in the living bodies and will sustain the collagen production by L-ascorbic acids for a relatively long period of time. Thus, the collagen-production enhancer stably sustains the collagen production of fibroblasts distributed in the dermis and other tissues and organs. The royal jellies act on keratinocytes to enhance the production of TGF-β and to promote proliferation of the cells, resulting in strengthening the skin and augmenting its biophylactic ability. As a result, when applied to the skin with age-related senility and the one damaged by factors such as ultraviolet ray, the collagen-production enhancer of the present invention has the following effects: It recovers the reduction of the collagen production of fibroblasts in the dermis, augments the biophylactic ability of the epidermis, imparts fitness to the skin, improves and prevents wrinkles, recovers the elasticity of the skin, and strengthens the tissues of viscera and veins. Since the collagen-production enhancer comprises L-ascorbic acids and royal jellies, it has not only an effect of enhancing the collagen production by L-ascorbic acids but also augments the systematic resistance of living bodies, early improves poor physical condition, effectively maintains healthy condition, improves skin roughness and its related skin disorders, and improves skin roughness and liable conditions thereto in healthy persons, which are all inherent to L-ascorbic acids and royal jellies, respectively. As described above, the compositions according to the present invention are useful in the prevention of senility of the skin and the maintenance of the desired healthy, wrinkleless skin; and it is also quite useful in foods/beverages, foods for special dietary uses, healthy functional foods, cosmetics, quasi-drugs, pharmaceuticals, feeds, baits for fish, pet foods, and daily goods that are directed to maintain and promote the beauty and the health. In addition, when used as cosmetics for applying to the skin including scalp, the compositions of the present invention effectively prevent skin disorders or improve the therapeutic effects on such disorders by conventional therapies, and exert a hair growth effect, etc., based on their above-identified functions and effects. When the collagen-production enhancer and/or the compositions containing the same according to the present invention are directly applied to the skin as cosmetics, they can be optionally accelerated to be penetrated into the skin by using the apparatus for iontophoresis disclosed in

Japanese Patent Application No. 2001-80195, titled "Iontophoresis". Since the action of enhancing the collagen production by L-ascorbic acids using royal jellies according to the present invention will be stably retained even after heated at temperatures of 70°C or higher for 30 min or longer, the compositions comprising the collagen-production enhancer of the present invention should not necessarily be cared for their stability during their processing and preservation. However, unheated royal jellies in themselves are susceptible to deterioration when in an intact form just after harvested and, as a problem, most of their useful actions tend to be lowered. To overcome such a problem, for example, International Patent Application No.WO 01/60388, based on Japanese Patent Application No. 2000-37200, titled "Cell Activator", discloses that the addition of α,α-trehalose to unheated royal jellies inhibits the deterioration of such royal jellies during their preservation at ambient temperature of about 25°C. Using compositions in a readily handleable form, the collagen-production enhancer of the present invention can be arbitrarily prepared.

The present invention is explained with reference to the following Experiments and Examples:

### Experiment 1: Study on the collagen production by L-ascorbic acids or royal jellies

### 1. L-Ascorbic acids

Sodium L-ascorbate, a special grade reagent commercialized by Wako Pure Chemical Industries, Ltd., Tokyo, Japan, was used as L-ascorbic acids.

### 2. Royal jellies

In this experiment, a heated royal jelly and an unheated royal jelly were used as royal jellies. The unheated royal jelly was prepared by thawing an intact royal jelly from Brazil, having a moisture content of 67% and being preserved at -20°C, at ambient temperature prior to use, and promptly portioning the resultant in a desired amount. The heated royal jelly was prepared by placing five gram aliquots of the unheated royal jelly in glass test tubes, 18 mm in diameter; heating the aliquots at 40, 50, 60, 70, 80 or 90°C for 30 min in a temperature-controlled bath or at 100° C for 30 min in an oil bath, followed by cooling them to 30°C or lower for use in the following experiments.

### 3. Preparation of new-born hamster fibroblasts

According to conventional manner, the dorsum skin of a new-born hamster was cut and detached, and fibroblasts were isolated from the detached skin. The preparation method is outlined below: To detach the epidermis from the dermis, the debris was placed and allowed to stand at 4°C overnight in Eagle's minimum essential medium, commercialized by Nippon Suisan Kaisha, Ltd., Tokyo, Japan, containing 0.03 mM Ca²⁺, in which 500 units/ml of a dispase commercialized by Godo Shusei Co., Ltd., Tokyo, Japan, was dissolved. The detached dermis was kept at 37°C for one hour in Dulbecco minimum essential medium (abbreviated as "D-MEM", hereinafter), commercialized by Nippon Suisan Kaisha, Ltd., Tokyo, Japan, in which 0.25% (v/v) of a collagenase commercialized by Amano Enzyme, Aichi, Japan, was dissolved. Thereafter, the resulting cell debris of the dermis were pipetted into a cell suspension with singly dispersed cells of new-born hamster fibroblasts, followed by collecting the cells by centrifugation. The cells were suspended in phosphate-buffered saline and allowed to stand for 30 min, followed by centrifugally collecting the resulting supernatant with fibroblasts and re-suspending the cells in D-MEM supplemented with 10% (v/v) of a fetal bovine serum (abbreviated as "FCS", hereinafter) commercialized by GIBCO Bethesda Research Laboratories, Bethesda, Maryland, USA, into a cell suspension for use in the following assay for collagen production level.

### 4. Assay for the level of collagen production by L-ascorbic acid

The following cells were cultured in an incubator with 5% (v/v) CO₂ at 37°C. Three milliliter aliquots of the cell suspension of new-born hamster fibroblasts, prepared in the above 3, were seeded in 6-well plates to give a cell density of 4 x 10⁵ cells/well and cultured for seven days. To D-MEM supplemented with 10% (v/v) FCS was added and dissolved therein L-ascorbic acid to give a concentration of 0.0, 0.1, 0.2, 0.5, 1.0, 2.0, 5.0, 10.0, 20.0, 50.0, 100.0, or 200.0 µg/ml. Similarly as above, either a heated royal jelly or an unheated royal jelly was added to and dissolved in D-MEM supplemented with 10% (v/v) FCS to give a concentration of 0.0, 0.1, 0.2, 0.5, 1.0, 2.0, 5.0, 10.0, 20.0, 50.0, 100.0, 200.0, or 500.0 µg/ml. Five milliliter aliquot of any one of the above media supplemented with L-ascorbic acid or the heated or unheated royal jelly was added to each well of the plates, which had been removed off the culture supenatants of fibroblast cultures, followed by culturing the cells for three days. Thereafter, the supernatant in each well was replaced with five milliliters of a fresh medium supplemented with the same concentration of L-ascorbic acid or the heated or unheated royal jelly as mentioned-above, and the cells were sequentially cultured for three days, replaced with one milliliter of a fresh medium having the same concentration of L-ascorbic acid or the heated or unheated royal jelly as mentioned-above, cultured for three hours, admixed with [2, 3-³H] proline (40 Ci/mmol) commercialized by Amersham Corp., Div. Amersham International, IL, USA, which had been dissolved in D-MEM to give a concentration of 3 µCi/well, and further cultured overnight. All the culture media, including those supplemented with L-ascorbic acid or the heated or unheated royal jelly, used in this experiment, were filtered with a 0.22 µm filter before use.

### 5. Quantitation of proline taken into collagen produced by fibroblasts

In the above 4, after the fibroblasts of new-born hamster were cultured in the presence of [2,3-³H] proline, collagen was extracted from the cells in a usual manner and quantified for [2,3-³H] proline taken thereinto. The quantitation was carried out by the method disclosed by Seong-Jin Kim et al. in Dermatologic Surgery, Vol. 24, pp. 1,054-1,058 (1998). The method is summarized in the below: To each well of the cell cultures, that had been cultured overnight with [2,3-³H] proline in the above 4 and removed their supernatants, was added 0.3 ml/well of a solution of trypsin commercialized by GIBCO Bethesda Research Laboratories, Bethesda, Maryland, USA, and the resulting cells were sequentially cultured at 37°C for 10 min, and admixed with and suspended in 0.3 ml/well of D-MEM. The cell suspensions were centrifuged to remove supernatants and to collect cells. The collected cells were admixed with 0.1 ml of 1M-acetic acid solution containing 1 mg/ml of a solution of pepsin commercialized by Sigma Chemical Co., MO, USA, and shaken for four hours. To the resulting each solution was added 0.8 ml of 0.5 M acetic acid solution containing 200 µg/ml of type I collagen, commercialized by Koken Co., Ltd., Tokyo, Japan, and centrifuged at 3,000 rpm/min at 4°C for five minutes, followed adding 5 M sodium chloride solution to the resulting each solution to give a sodium chloride concentration of 0.15 M, and centrifuging the solution at 12,000 rpm at 4°C for 10 min. The resulting supernatant of each cell culture was adjusted to 0.45 M sodium chloride solution using 5 M sodium chloride solution and centrifuged at 3,200 rpm and 4°C for 30 min to remove the supernatant. Finally, 0.25 ml of 0.5 M acetic acid solution was added to the resulting sediment of each cell culture, and the mixture was stirred for suspending and then further suspended in 5 ml of a scintillation solution before quantifying the ³H proline, taken into collagen, by a liquid scintillation counter in a usual manner. In this experiment, it was conducted with three wells for each concentration of sodium L-ascorbate or the heated or unheated royal jelly.

Table 1 shows the result of the relationship between the amount of L-ascorbic acid added and the amount of collagen produced by new-born hamster fibroblasts in terms of a relative value, regarding the collagen production level or the count number by the liquid scintillation counter of a cell culture system with no addition of L-ascorbic acid as 100. In the case of adding no sodium L-ascorbate, new-born hamster fibroblasts produced a relatively low level of collagen; while in the case of adding sodium L-ascorbate, the fibroblasts showed an increased collagen production level depending on the concentration of sodium L-ascorbate, confirming the collagen production by L-ascorbic acid. This experiment system revealed that a minimum effective amount of L-ascorbic acid for producing collagen was 0.5 µg/ml, and that the level of collagen production reached a plateau level and no significant increase was observed at concentrations of 50 µg/ml or higher. Although concrete data is not shown, royal jelly exhibited no enhancement of collagen production when used alone at any concentrations tested in this experiment, independently of the use of heated or unheated royal jelly.

**Table 1**

| Concentration of L-ascorbic acid | Relative amount of collagen production* |
|---|---|
| (µg/ml) | |
| 0.0 | 100±12 |
| 0.1 | 95±13 |
| 0.2 | 118±26 |
| 0.5 | 210±33** |
| 1.0 | 482±89** |
| 2.0 | 1497±155** |
| 5.0 | 1755±151** |
| 10.0 | 2089±160** |
| 20.0 | 2456±186** |
| 50.0 | 2841±341** |
| 100.0 | 2956±500** |
| 200.0 | 2863±456** |

| | |
|---|---|
| Note * : (Relative value) ± (Standard deviation) ** : There exists a significant difference (p<0.05). | |

### Experiment 2: (1) Study on the influence of royal jellies on the collagen production by L-ascorbic acids

Using the assay system for the collagen production level used in Experiment 1, it was studied the influence of royal jellies on the collagen production by L-ascorbic acids: New-born hamster fibroblasts, prepared similarly as the method in Experiment 1, were seeded in 6-well plates and cultured for seven days. The supernatant in each plate was removed, followed by adding to each plate 5 ml/well of a medium, which had been prepared by dissolving sodium L-ascorbate to give a concentration of 0.0, 0.1, 0.2, 0.5, 1.0, 2.0, 5.0, 10.0, 20.0, 50.0, or 100.0 µg/ml in terms of L-ascorbic acid in D-MEM supplemented with 10% (v/v) FCS, and dissolving heated or unheated royal jelly in the resulting each medium to give a concentration of 0.0, 2.0, 5.0, 10.0, 20.0, 50.0, 100.0, 200.0, or 500.0 µg/ml; and assaying the collagen production level similarly as the method in Experiment 1. In this experiment, three wells were used for each concentration. The results were evaluated whether there existed a significant difference between the count in a control system and those in the test systems with different concentrations of heated or unheated royal jelly, based on the count for the control system with no royal jelly that was detected by a liquid scintillation counter, as a control. In the significant difference test, the enhancement of royal jelly on the collagen production by L-ascorbic acid was judged based on the criterion with the symbols of "+", "±", and "-", meaning significant errors of p<0.05, 0.1>p>0.05, and p>0.1, respectively. The fibroblasts with at least 50 µg/ml of L-ascorbic acid reached a plateau level and gave no more enhancement within the range of royal jelly tested in this experiment. Since there were found no significant difference between the systems with heated royal jelly and those with unheated royal jelly, which had been treated at a temperature of 40, 50, 60, 70, 80, 90, or 100°C, in terms of their actions of enhancing the collagen production by L-ascorbic acid, only the data on the unheated royal jelly and L-ascorbic acid with concentrations in the range of 0 to 20 µg/ml was shown in Table 2.

**Table 2**

| | Concentration of L-ascorbic acid (µg/ml) | | | | | | | | | |
|---|---|---|---|---|---|---|---|---|---|---|
| | | 0.0 | 0.1 | 0.2 | 0.5 | 1.0 | 2.0 | 5.0 | 10.0 | 20.0 |
| | 0.0 | - | - | - | - | - | - | - | - | - |
| | 2.0 | - | - | - | - | - | - | - | - | - |
| Concentration of royal jelly | 5.0 | - | - | - | - | - | - | - | ± | ± |
| | 10.0 | - | - | - | ± | + | + | + | + | + |
| (µg/ml) | 20.0 | - | - | ± | + | + | + | + | + | + |
| | 50.0 | - | - | + | + | + | + | + | + | + |
| | 100.0 | - | - | + | + | + | + | + | + | + |
| | 200.0 | - | - | + | + | + | + | + | + | + |
| | 500.0 | - | - | + | + | + | + | + | + | + |

As shown in Experiment 1, the sole use of L-ascorbic acid could exert the collagen-production action only when used at concentrations of 0.5 µg/ml or more, but it becomes to exert the action even at a concentration as low as 0.2 µg/ml when used in combination with royal jelly. Considering the fact that the royal jelly used in this experiment did not contain L-ascorbic acid, the above result showed that royal jelly contains an ingredient(s), other than L-ascorbic acid, that enhances the ability of enhancing the collagen production by L-ascorbic acid; and has an action of lowering the concentration of L-ascorbic acid requisite for exerting the collagen production action as compared with that with the sole use of L-ascorbic acid. In addition, it was confirmed that, when 20.0 µg/ml of L-ascorbic acid is used and at least 10 µg/ml of royal jelly is added, royal jelly enhances the collagen production by L-ascorbic acid. The fact shows that, when used in combination, royal jellies enhance the collagen production by L-ascorbic acid and stably retain the collagen production ability of L-ascorbic acid even when L-ascorbic acids are used alone and decomposed *in vivo* up to a reduced concentration level where L-ascorbic acids could not exert their inherent collagen production action or could merely exert a reduced collagen production ability.

### Experiment 3: (2) Study on the influence of royal jellies on the collagen production by L-ascorbic acids

In a system with 200 µg/ml of royal jelly where the enhancement of collagen production by L-ascorbic acid was observed in Experiment 2, the influence of royal jelly on the collagen production by L-ascorbic acid or L-ascorbic acid 2-glucoside was examined.

### (1) L-Ascorbic acids:

Sodium L-ascorbate, the same specimen as used in Experiment 1, and L-ascorbic acid 2-glucoside commercialized by Hayashibara Biochemical Laboratories Inc., Okayama, Japan, were used as L-ascorbic acids.

### (2) Royal jellies:

The royal jelly used in this experiment was the same specimen of unheated royal jelly as used in Experiment 1.

### (3) Assay for the collagen production by sodium L-ascorbate or L-ascorbic acid 2-glucoside:

Royal jelly was dissolved in D-MEM medium containing 10% (v/v) FCS to give a concentration of 200 µg/ml, and then L-ascorbic acid 2-glucoside was dissolved in the resulting medium to give a concentration of 0.2, 0.5 or 1.0 µg/ml in terms of the weight of L-ascorbic acid. As controls, L-ascorbic acid 2-glucoside was dissolved in D-MEM medium, supplemented with 10% (v/v) FCS but with no royal jelly, to give a concentration of 0.2, 0.5, or 1.0 µg/ml in terms of the weight of L-ascorbic acid. Similarly as in Experiment 1, new-born hamster fibroblasts were cultured for seven days in 6-well plates and further cultured for seven days after the supernatant in each plate was replaced with 5 ml of any of the above media, which dissolved the above-identified L-ascorbic acid 2-glucoside and royal jelly, or those prepared for use as controls. Thereafter, the collagen production level in each well was determined by the same method as in Experiment 1. As a negative control, a medium, supplemented with 200 µg/ml of royal jelly and 1 µg/ml of sodium L-ascorbate in terms of the weight of L-ascorbic acid, was added to the fibroblasts similarly as above, and the cells were cultured for seven days.

The results are in Table 3 which shows the relationship between the amount of L-ascorbic acid 2-glucoside added in the presence or absence of royal jelly and the collagen production level of new-born hamster fibroblasts, based on a criterion that the collagen production level of the fibroblasts was regarded as 100 for a system with no L-ascorbic acids and royal jellies. The fibroblasts with no addition of L-ascorbic acid 2-glucoside gave a lower collagen production level similarly as that of the control in Experiment 1, independently of the addition of royal jellies. When ascorbic acid 2-glucoside was added to give a concentration of 0.2 µg/ml or more, the collagen production level was enhanced as compared with the system with no L-ascorbic acid 2-glucoside, and the level was more enhanced by the addition of royal jellies. Although concrete data are omitted, the fibroblasts gave an enhanced level of collagen production when cultured for three days after the addition of 1 µg/ml of L-ascorbic acid and further cultured for three days after the culture medium was replaced with a fresh preparation of the same medium. While the negative control showed no enhancement of collagen production because, even if cultured in a medium supplemented with 1 µg/ml of L-ascorbic acid, the fibroblasts were not cultured by replacing the culture medium with a fresh one after 3-day culture. The fact shows that L-ascorbic acid 2-glucoside, dissolved in media, was advantageously more stable as compared with L-ascorbic acid and was gradually hydrolyzed by intracellular glucosidase into L-ascorbic acid and glucose to durably exert the activity of L-ascorbic acid for seven days, indicating that L-ascorbic acid 2-glucoside has an advantage when royal jellies are used in combination with L-ascorbic acids.

**Table 3**

| Concentration of L-ascorbic acid 2-glucoside (µg/ml) | Addition of royal jelly (200 µg/ml) | Collagen production level* |
|---|---|---|
| 0.0 | Non | 100±23 |
| | Yes | 134±34 |
| 0.2 | Non | 219±31 |
| | Yes | 298±30** |
| 0.5 | Non | 527±17 |
| | Yes | 1440±70** |
| 1.0 | Non | 2250±436 |
| | Yes | 3168±318** |

| | | |
|---|---|---|
| *: (Relative value) ± (Standard deviation) **: There exists a significant difference (p<0.05) from the system with an equal concentration of L-ascorbic acid 2-glucoside but with not royal jelly. | | |

### Experiment 4 : Study of the influence of royal jelly and/or L-ascorbic acid 2-glucoside on the production of TGF-β by fibroblasts

To study the mechanism of the enhancement of collagen production by royal jellies and/or L-ascorbic acids, the influence of royal jelly and/or L-ascorbic acid 2-glucoside, which had been reported to enhance the production of mRNA for collagen in fibroblasts, on the production of TGF-β was examined by the following method:
(1) Assay for TGF-β:
   TGF-β was assayed on TGF-β1 Emax ImmunoAssay System, commercialized by Promega Corporation, Tokyo, Japan.
   (2) Assay for TGF-β produced by fibroblasts in the presence of royal jelly and/or L-ascorbic acid 2-glucoside:
      TGF-β was assayed by inoculating 1x10⁴ cells/well of new-born hamster fibroblasts, prepared by the method similarly as in Experiment 1, to 96-well microplates, commercialized by Nippon Becton Dickinson, Company, Ltd., Tokyo, Japan; culturing the cells to proliferate into confluent cells growing over the bottom surface of each well; removing the supernatant in each well; adding to each plate 200 µl/well of a D-MEM medium (I) supplemented with 10% (v/v) FCS, another medium of the D-MEM medium (I) with or without 0.2 µg/ml L-ascorbic acid 2-glucoside in terms of the weight of L-ascorbic acid and with or without royal jelly dissolved to give a concentration of 200 µg/ml, or the other medium of the D-MEM medium (I) dissolving 200 µg/ml of royal jelly; culturing the cells for 24 hours; and quantifying the TGF-β produced in each supernatant.

As a result, new-born hamster fibroblasts produced about 300 picograms (abbreviated as "pg" hereinafter) of TGF-β per milliliter even in the absence of L-ascorbic acid 2-glucoside and royal jelly. The data is in Table 4 where the TGF-β production level of the fibroblasts is expressed with a relative value, when the TGF-β production level of the cells in the absence of L-ascorbic acid 2-glucoside and royal jelly is regarded as 100. No change was found in the TGF-β production level of the fibroblasts by the addition of 200 µg/ml of royal jelly. When cultured by the addition of the culture medium supplemented with 0.2 µg/ml of L-ascorbic acid 2-glucoside in terms of the weight of L-ascorbic acid, the TGF-β production level tended to be enhanced compared with the system with no addition of L-ascorbic acid 2-glucoside, and the production level of which was significantly enhanced by the addition of 200 µg/ml of royal jelly. The enhancement of TGF-β production well correlated with the enhancement of the collagen production by L-ascorbic acid 2-glucoside induced by royal jelly as shown in Experiment 3, suggesting that there exists a mechanism that enhances the production of TGF-β, among the mechanisms of the enhancement of the collagen production by L-ascorbic acids using royal jellies.

**Table 4**

| Concentration of L-ascorbic acid 2-glucoside (µg/ml) | Addition of royal jelly (200 µg/ml) | Production level* of TGF-β |
|---|---|---|
| 0.0 | No | 100±9 |
| | Yes | 98±9 |
| 0.2 | No | 111±5 |
| | Yes | 132±12** |

| | | |
|---|---|---|
| * : (Relative value) ± (Standard deviation) ** : There exists a significant difference (p<0.05) from the system with an equal concentration of L-ascorbic acid 2-glucoside but with no royal jelly. | | |

### Experiment 5

### Study of the influence of royal jelly on the TGF-β production by keratinocyte

Confirmation of the fact that royal jelly enhances the production of TGF-β of fibroblasts by L-ascorbic acids led to a speculation that keratinocytes in the epidermis adjacent to the dermis where fibroblasts exist, would possibly influence on fibroblasts, and also led to a study of the influence of royal jelly and/or L-ascorbic acid 2-glucoside on the TGF-β production by keratinocytes, according to the following methods:

### (1) Culture medium for keratinocytes:

To culture keratinocytes, the following culture medium, comprising HKGS as a proliferation additive and Epilife^{®} commercialized by Cascade Biologics, Inc., OR, USA, was used as a basal culture medium for the cells: The medium was prepared by adding to Epilife^{®} medium 0.06 mM Ca²⁺; HKGS in a sufficient amount that finally gave concentrations of 5 µg/ml of calf insulin, 5 µg/ml of calf transferrin, 5 µM hydrocortisone, and 0.2% of an extract of calf hypophysis, commercialized by Cascade Biologics Inc., USA; 100 units/ml of penicillin commercialized by Meiji Seika Kaisha Ltd., Tokyo, Japan; and 100 µg/ml streptomycin commercialized by Meiji Seika Kaisha, Ltd., Tokyo, Japan.

### (2) Preparation of keratinocytes of new born hamster:

The dermis was detached from a dorsal skin fragment of a 4-day-old new-born hamster, prepared similarly as in Experiment 1, cut into pieces with metal tongs and centrifuged to collect precipitates, to which was then added 20 units/ml of DNase commercialized by Sigma Chemical Company, MO., USA. The resulting mixture was gently stirred for three minutes at ambient temperature, admixed with the standard MEM medium (abbreviated as "S-MEM" hereinafter) commercialized by Nissui Pharmaceutical Co., Ltd., Tokyo, Japan, stirred for two minutes, and centrifuged to collect cells, followed by suspending the cells in S-MEM. The following cell cultures were carried out at 37°C in an incubator with an atmospheric condition of 5% (v/v) CO₂.

### (3) Assay for the enhanced level of TGF-β production by royal jelly:

The keratinocytes suspended in S-MEM were collected by centrifugation, re-suspended in a basal culture medium for keratinocytes, and seeded in Type IV collagen-coated 96-well microplates commercialized by Nippon Becton Dickinson Company, Ltd., Tokyo, Japan, to give a cell concentration of 1x10⁴ cells/100 µl/well. After the cells adhered to the bottom surfaces of the wells, each culture medium in each well was replaced with a test solution prepared by dissolving a fresh preparation of the same royal jelly as used in Experiment 1 in the basal culture medium for keratinocytes to give a concentration of 1,000 µg/ml, or another test solution prepared by stepwisely diluting the above culture medium by two folds in series with the basal culture medium for keratinocytes to give a concentration of 500, 250, 125, 62.5 or 31.3 µg/ml of royal jelly. After three days incubation, each test solution in each well was replaced with a fresh preparation of the same test solution used, and the keratinocytes were cultured for 24 hours. Thereafter, TGF-β in each supernatant was quantified by the TGF-β1 Emax ImmunoAssay System, similarly as in Experiment 4. The keratinocytes in the system with no L-ascorbic acids and royal jellies produced about 70 pg/ml of TGF-β. Table 5 shows the result of this experiment, where the values are expressed with relative values when the TGF-β production level for the system with no addition of L-ascorbic acids and royal jellies is regarded as 100.

### (4) Assay for the proliferation enhancement of keratinocytes by royal jelly:

Keratinocytes were incubated under the same conditions and for the same period as in Experiment 3, and then sequentially incubated for another three days, admixed with 20 µl/well of "ALAMER BLUE^{™}" commercialized by Trek Diagonostic Systems Ltd., Ohio, USA, incubated at 37°C for three hours, and subjected to measurement for fluorescent intensity on "FLUOROSKAN II^{™}", Labosystems, Japan, Inc., Tokyo, Japan, at an excitation wavelength of 544 nm and a fluorescent wavelength of 590 nm. Table 6 shows the results where the values are expressed with relative values based on the criterion that the amount of keratinocytes, i.e., the fluorescent intensity, for the culture incubated in the absence of royal jelly is regarded as 100.

Royal jelly enhanced the production level of TGF-β in keratinocytes at concentrations of 125 µg/ml or over depending on the concentrations, and the enhancement was particularly distinct at concentrations of 500 µg/ml or over. The result indicates that royal jelly acts on not only fibroblasts but keratinocytes, adjacent to the dermis where fibroblasts exist, to enhance the production of TGF-β, the TGF-β produced by both of the fibroblasts and the keratinocytes enhances the collagen production by the fibroblasts, and that royal jelly acts on the keratinocytes in the epidermis before reaching the fibroblasts. Thus, the effects of royal jelly will be exerted effectively and smoothly. Although concrete data is not shown, L-ascorbic acids gave no influence on the TGF-β production and the proliferation of keratinocytes, independently of the presence or the absence of royal jelly.

**Table 5**

| Concentration of royal jelly | TGF-β production level* |
|---|---|
| (µg/ml) | |
| 0.0 | 100±13 |
| 31.3 | 110±18 |
| 62.5 | 113±28 |
| 125.0 | 197±18 |
| 250.0 | 223±20 |
| 500.0 | 532±162** |
| 1000.0 | 734±230** |

| | |
|---|---|
| Note * : (Relative value) ± (Standard deviation) ** : It has a significant difference (p<0.05) compared with a system with no royal jelly. | |

Royal jelly exhibited a proliferation enhancement of keratinocytes at concentrations of 62.5 µg/ml or over depending on its concentration, and the enhancement level was particularly distinct at concentrations of 250 µg/ml or over. The result indicates that royal jelly has no or less cytotoxicity, and that the collagen-production enhancer also has an action of strengthening biodefence ability inherent to the skin.

**Table 6**

| Added amount of royal jelly | Proliferation level of keratinocyte* |
|---|---|
| (µg/ml) | |
| 0.0 | 100±6 |
| 31.3 | 102±7 |
| 62.5 | 115±15 |
| 125.0 | 114±5 |
| 250.0 | 128±7** |
| 500.0 | 138±3** |
| 1000.0 | 149±10** |

| | |
|---|---|
| Note * : (Relative value) ± (Standard deviation) ** : It has a significant difference (p<0.05) compared with a system with no royal jelly. | |

### Experiment 6

### Safety test on collagen-production enhancer

Needless to say that the material royal jellies and L-ascorbic acids used in the collagen-production enhancer of the present invention are highly safe because they have been widely used in the fields of health foods and cosmetics, however, the safeness of the enhancer was reconfirmed by the test using mice: To one part by weight of sodium L-ascorbate, in terms of L-ascorbic acid, as used in Experiment 1, was added four parts by weight of a fresh preparation of the same unheated royal jelly or one of the same royal jelly heated at 100°C for 30 min, and the mixtures were diluted with an equal volume of deionized water into test specimens. As a control, deionized water was used as a control specimen. To a group of mice consisting of five DDY male mice, 5-week-old, with an average body weight of 25 g, commercialized by Charles River Japan, Inc., Tokyo, Japan, were orally administered 10 g/kg body weight/day, in terms of 5 g/kg of the collagen-production enhancer, of any of the test and control specimens for successive 30 days, while they were weighed and macroscopically observed their conditions. Considering the stability of L-ascorbic acid, the test specimens were prepared daily just before their administrations.

All the mice in the test group, which were orally administered with the collagen-production enhancer containing sodium L-ascorbate and heated or unheated royal jelly, gained weight and had healthy conditions similarly as the mice in the control group. The data from this experiment revealed that the collagen-production enhancer of the present invention, which comprises L-ascorbic acids and royal jellies, has a relatively high safeness.

The following Examples explain the present invention in more detail but they do not limit the scope of the present invention.

### Example 1

### Collagen-production enhancer

The following ingredients were mixed to homogeneity according to the ratio as indicated below to obtain a collagen-production enhancer. In accordance with the methods in Experiments 1, 4 and 5, the product was diluted with an appropriate medium for assaying each activity, confirming that the product has the actions of producing collagen and TGF-β, and promoting the proliferation of keratinocyte.

| | |
|---|---|
| Sodium L-ascorbate | 1.0 part by weight |
| Unheated royal jelly used in Experiment 1 | 20.0 parts by weight |

The product is a readily usable, effective collagen-production enhancer which exerts an action of enhancing the collagen production by L-ascorbic acid, moisturizes the skin, and prevents aging of the skin. Since the product has a satisfactory taste due to its adequate sour taste, it can be used as a daily usable health food and also as a TGF-β production enhancer or a keratinocyte proliferation promotor. The product can be arbitrarily, orally taken intact or after dissolved in water or other beverages. When incorporated into foods for special dietary uses, health-promoting foods, cosmetics, quasi-drugs, pharmaceuticals, feeds, baits for fish, pet foods, and daily goods, the collagen production enhancer arbitrarily imparts them advantageous actions of collagen production, TGF-β production enhancement, and/or keratinocyte proliferation promotion.

### Example 2

### Health food

The following ingredients were mixed to homogeneity according to the ratio as indicated below to obtain a collagen-production enhancer which was then dried *in vacuo* at ambient temperature overnight, and processed into a powdery collagen-production enhancer using a pulverizer. The enhancer was diluted with D-MEM supplemented with 10% (v/v) FCS and, in accordance with Experiment 1, assayed for the activity of enhancing collagen production, confirming that the product exerted the desired activity.

| | |
|---|---|
| L-Ascorbic acid 2-glucoside commercialized by Hayashibara Biochemical Laboratories, Inc., Okayama, Japan | 1.0 part by weight |
| | |
| Unheated royal jelly used in Experiment 1 | 10.0 parts by weight |
| | |
| "TREHA^{™}", a hydrous crystalline trehalose commercialized by Hayashibara Shoji, Inc. Okayama, Japan | 38.0 parts by weight |

The enhancer can be optionally admixed with sodium hydroxide to adjust its pH to meet its use.

The enhancer is a readily usable, effective collagen-production enhancer which continuously exerts an action of enhancing collagen production. Since the enhancer has a satisfactory taste due to its mild sweetness and adequate sour taste, does not cause browning, and has a relatively long shelf-life, it can be used as a daily usable health food. Also the enhancer can be arbitrarily used as an orally or intubationally administrable composition for animals such as domestic animals and pets, as well as for humans; or used for breed animals such as fish, shrimps, and lobsters directly or after mixed with conventional feeds.

Sucrose fatty acid ester was added to the above enhancer to give a concentration of one percent by weight and tabletted with a tabletting machine into tablets, about 300 mg each. The product is an easily, orally administrable, highly effective collagen production enhancer, which continuously exerts an action of enhancing collagen production and has a relatively long shelf-life and advantageous transportability.

### Example 3

### Health food

The following ingredients were mixed to homogeneity according to the ratio as indicated below and dried *in vacuo* to obtain a collagen-production enhancer in the form of a paste, confirming that the product stably exerts the desired activity of enhancing collagen production.

| | |
|---|---|
| Sodium L-ascorbate | 1.5 parts by weight |
| | |
| L-ascorbic acid | 1.0 part by weight |
| | |
| Heated royal jelly prepared by heating the same unheated royal jelly used in Experiment 1 at 70°C for 30 min | 20.0 parts by weight |
| "αG RUTIN^{™}", a glycosyl rutin commercialized by Hayashibara Shoji, Inc. Okayama, Japan | 1.0 part by weight |
| | |
| Anhydrous crystalline maltitol | 1.5 parts by weight |

The product is an easily usable, effective collagen-production enhancer, which stably exerts an action of enhancing collagen production. Also the product is useful as a daily usable health food because it exerts a satisfactory action of enhancing collagen production, has a mild sweetness and an adequate acid taste suitable for imparting a satisfactory taste, and exerts an advantageous effect on the normalization of the skin tissue and the prevention of aging of the skin tissues.

### Example 4

### Ice cream

A mixture of 18 parts by weight of a raw cream containing about 46% by weight of fat and oil, seven parts by weight of a skim milk powder, 51 parts by weight of a total milk, 13 parts by weight of sucrose, two parts by weight of pullulan, and one part by weight of guar gum was melted and sterilized by keeping at 70°C for 30 min, emulsified, dispersed by a homogenizer into a suspension, and instantly cooled to 3 to 4°C. To the resulting mixture was added five parts by weight of a collagen-production enhancer obtained by the method in Example 2, mixed, aged overnight, and freezed by a freezer to obtain an ice cream.

The product is an ice cream which has an adequate sweetness and a high quality flavor and taste, exerts an action of enhancing collagen production, moisturizes the skin, and effectively prevents aging of the skin.

### Example 5

### Fruit jelly

| | |
|---|---|
| Sucrose | 14.0 parts by weight |
| Hydrous crystalline trehalose | 2.0 parts by weight |
| Gelatin | 2.5 parts by weight |
| Grape fruit juice | 32.0 parts by weight |
| Water | 43.5 parts by weight |
| | |
| Heated royal jelly prepared by heating the same unheated royal jelly used in Experiment 1 at 70°C for 30 min | 4.0 parts by weight |
| | |
| L-Ascorbic acid | 2.0 parts by weight |

Sucrose, trehalose, and gelatin were added to water and dissolved therein by heating at 95°C. To the resulting solution was added the grape fruit juice, sterilized by heating at 80°C for 30 min, and admixed with the L-ascorbic acid and the heated royal jelly to obtain a fruit jelly.

The product is a fruit jelly which has an adequate sweetness and smooth texture, exerts an action of enhancing collagen production, prevents aging of the skin, and effectively maintains and promotes the beauty and the health.

### Example 6

### Health beverage

A composition was prepared by mixing 500 parts by weight of anhydrous crystalline maltose, 100 parts by weight of the collagen-production enhancer obtained in Example 2, 190 parts by weight of a powdered egg yolk, 200 parts by weight of a skim milk powder, 4.4 parts by weight of sodium chloride, 1.85 parts by weight of potassium chloride, four parts by weight of magnesium sulfate, 0.01 part by weight of thiamine, 0.6 part by weight of vitamin E acetate, 0.04 part by weight of nicotinic acid amid, and 0.02 part by weight of "αG HESPERIDIN PS^{™}", a glycosyl hesperidin. Twenty-five parts by weight of the composition were homogeneously dispersed and dissolved in 150 parts by weight of refined water, and 200 g aliquots of the resulting solution were injected into and sealed in brown glass vials.

Since the product continuously exerts an action of enhancing collagen production and has supplemental nutritions, it can be arbitrarily used as a health beverage directed to improve the beauty and the health. The product can be also used as an orally or intubationally administrable composition for domestic and pet animals, as well as humans.

### Example 7

### External dermatological cream

The composition (1) as indicated below was admixed with the composition (2) in a usual manner, and the mixture was cooled to 30°C or lower and further mixed with the collagen production enhancer as indicated below, adjusted to give a slightly acid pH with potassium hydroxide, and emulsified with a homogenizer to obtain an external dermatological cream.

| Composition (1): | |
|---|---|
| Polyoxyethylene glyceryl monostearate | 2.0 parts by weight |
| Glyceryl monostearate, self-emulsifying | 5.0 parts by weight |
| Eicosanyl behenate | 1.0 part by weight |
| Liquid paraffine | 1.9 parts by weight |
| Trimethylolpropane trioctanoate | 10.0 parts by weight |
| | |
| Composition (2): | |
| 1,3-Butylene glycol | 5.0 parts by weight |
| Sodium lactate solution | 10.0 parts by weight |
| Ginseng extract | 1.5 parts by weight |
| Methyl parahydroxybenzoate | 0.1 part by weight |
| Sodium hyaluronate | 0.1 part by weight |
| Licorice extract | 0.5 parts by weight |
| Refined water | 62.4 parts by weight |
| | |
| Collagen production enhancer: | |
| L-Ascorbic acid 2-glucoside commercialized by Hayashibara Biochemical Laboratories, Inc., Okayama, Japan | 2.0 parts by weight |
| | |
| Unheated royal jelly used in Experiment 1 | 3.0 parts by weight |

The cream thus obtained can be optionally prepared into a slightly alkaline cream by the addition of potassium hydroxide.

The product is useful as a basic skin care because it continuously enhances the collagen production in the skin through the enhancement of TGF-β production in fibroblasts and keratinocytes for promoting the proliferation of keratinocytes and keeping the moisture level of the skin, improving the skin fitness and sagging, exerting effect on wrinkles including finely ones, preventing aging of the skin, and exerting an effective moisturizing ability.

### Example 8

### TGF-β Production enhancer

The following ingredients were mixed to homogeneity according to the ratio as indicated below, allowed to stand at ambient temperature overnight, and prepared into a powdery TGF-β production enhancer using a pulverizer. The product thus obtained was diluted with a fresh preparation of the same culture medium for culturing keratinocytes used in Experiment 5 and, in accordance with Experiment 5, the dilute was assayed for activity of enhancing TGF-β production and promoting keratinocyte proliferation, and confirmed to exert these activities.

| | |
|---|---|
| Unheated royal jelly used in Experiment 1 | 1.0 part by weight |
| | |
| "FINETOSE^{™}", an anhydrous crystalline maltose commercialized by Hayashibara Shoji, Inc., Okayama, Japan | 49.0 parts by weight |

Since the product acts on keratinocytes to enhance their TGF-β production and promote their proliferation and this results in an enhancement of their collagen production, it is an advantageous TGF-β production enhancer which moisturizes the skin, prevents aging of the skin, and has an easy handleability and satisfactory efficacy. The product can be suitably used as a daily usable health food because of its preferable taste due to its adequate sour taste. Also the product can be arbitrarily administered orally alone or after dissolved in water or other beverages. In addition, the product can be incorporated into foods for special dietary uses, healthy functional foods, cosmetics, quasi-drugs, pharmaceuticals, feeds, baits for fish, pet foods, and daily goods in order to impart the actions of enhancing the production of collagen, enhancing the production of TGF-β, and/or promoting the proliferation of keratinocytes.

### Industrial Applicability

As explained above, the present invention was made based on a complete self-finding that the collagen-production enhancer, which comprises L-ascorbic acids and royal jellies; exerts a significant action of enhancing the collagen production by L-ascorbic acids, that is inherently induced by unheated royal jelly; and has a satisfactory continuity of the action. Since the collagen-production enhancer of the present invention has the actions of enhancing the TGF-β production and the promotion of keratinocyte proliferation but has no fear of causing serious side effects, it can be easily and comfortably used for preventing the aging of the skin and maintaining/promoting the beauty and the health. By combining with other ingredients, the collagen-production enhancer of the present invention, having the above-identified advantageous features, it can be arbitrarily used in the form of a food product, beverage, food for special dietary uses, healthy functional food, cosmetic, quasi-drug, pharmaceutical, feed, bait for fish, pet food, daily good, or the like.

The present invention with such outstanding functions and effects is a significant invention that greatly contributes to this art.

## Claims

1. A composition for enhancing the production of collagen or TGF-β in fibroblast, which comprises L-ascorbic acid or derivative thereof in an amount of at least 0.02% by weight and royal jelly in an amount of at least 0.5 part by weight to one part by weight of said L-ascorbic acid or said derivative thereof.

2. The composition of claim 1, which further comprises one or more members selected from the group consisting of antioxidants, viscosity-imparting agents, saccharides and sugar alcohols.

3. The composition of claim 1, wherein said L-ascorbic acid derivative is an L-ascorbic acid 2-glycoside.

4. The composition of claim 1, wherein said royal jelly has been heated at 70°C or higher for at least 30 min.

5. The composition of claim 1, which is a food product, food for special dietary uses, healthy functional food, cosmetic, quasi-drug, pharmaceutical, feed, bait for fish, or pet food.

6. Use of the composition of claim 1 for the manufacture of a medicament for enhancing the production of collagen.

7. Use of royal jelly for the manufacture of a medicament for enhancing TGF-β production.

8. Use of royal jelly for the manufacture of a medicament for promoting the proliferation of keratinocytes.

9. Use of royal jelly for the manufacture of a medicament for enhancing the production of collagen or TGF-β in fibroblasts by L-ascorbic acid or a derivative thereof.

## Patentansprüche

1. Zusammensetzung zur Erhöhung der Produktion von Collagen oder TGF-β in den Fibroblasten, wobei die Zusammensetzung L-Ascoxbinsäure oder ein Derivat davon zu einem Anteil von mindestens 0.02 Gewichtsprozent und Gelée-Royale zu einem Anteil von mindestens 0.5 Gewichtsprozent auf ein Gewichtsanteil der L-Asccrbinsäure oder dem Derivat davon umfasst.

2. Zusammensetzung nach Anspruch 1, wobei die Zusammensetzung zusätzlich eines oder mehrere Mitglieder, ausgewählt aus der Gruppe bestehend aus Antioxidantien, Viskosität verleihenden Mitteln, Saccharide und Zuckeralkohole, umfasst.

3. Zusammensetzung nach Anspruch 1, wobei das L-Ascorbinsäurederivat ein L-Ascorbinsäure-2-Glykosid ist.

4. Zusammensetzung nach Anspruch 1, wobei das Gelée-Royale für mindestens 30 Minuten auf 70 °C oder mehr erwärmt wurde.

5. Zusammensetzung nach Anspruch 1, wobei die Zusammensetzung ein Lebensmittelprodukt, ein Lebensmittel für spezielle Diätanwendungen, ein funktionelles Gesundheitslebensmittel, ein Kosmetikum, ein arzneimittelähnlicher Stoff, ein Arzneimittel, Futter, Fischköder oder Tiernahrung ist.

6. Verwendung der Zusammensetzung nach Anspruch 1 zur Herstellung eines Medikaments zur Erhöhung der Produktion von Collagen.

7. Verwendung von Gelée-Royale zur Herstellung eines Medikaments zur Erhöhung der TGF-β-Produktion.

8. Verwendung von Gelée-Royale zur Herstellung eines Medikaments zur Förderung des Wachstums von Keratinozyten.

9. Verwendung von Gelee-Royale zur Herstellung eines Medikaments zur Erhöhung der Produktion von Collagen oder TGF-β in den Fibroblasten durch L-Ascorbinsäure oder einem Derivat davon.

## Revendications

1. Composition pour augmenter la production de collagène ou de TGF-β dans les fibroblastes, qui comprend de l'acide L-ascorbique ou un de ses dérivés en une quantité d'au moins 0,02 % en poids, et de la gelée royale en une quantité d'au moins 0,5 partie en poids pour une partie en poids dudit acide L-ascorbique ou dudit dérivé de celui-ci.

2. Composition suivant la revendication 1, qui comprend en outre un ou plusieurs membres choisis dans le groupe consistant en des antioxydants, des agents conférant une viscosité, des saccharides et des sucres-alcools.

3. Composition suivant la revendication 1, dans laquelle ledit dérivé d'acide L-ascorbique est un 2-glycoside d'acide L-ascorbique.

4. Composition suivant la revendication 1, dans laquelle ladite gelée royale a été chauffée à une température égale ou supérieure à 70°C pendant au moins 30 minutes.

5. Composition suivant la revendication 1, qui est un produit alimentaire, un aliment pour des utilisations diététiques particulières, un aliment fonctionnel de santé, un produit cosmétique, un quasi-médicament, un agent pharmaceutique, un aliment pour animaux, un appât pour poissons ou un aliment pour animaux de compagnie.

6. Utilisation de la composition suivant la revendication 1, pour la production d'un médicament destiné à augmenter la production de collagène.

7. Utilisation de gelée royale pour la production d'un médicament destiné à augmenter la production de TGF-β.

8. Utilisation de gelée royale pour la production d'un médicament destiné à favoriser la prolifération des kératinocytes.

9. Utilisation de gelée royale pour la production d'un médicament destiné à augmenter la production de collagène ou de TGF-β dans les fibroblastes par l'acide L-ascorbique ou un de ses dérivés.
